# DEMANDE DE BREVET EUROPEEN

(11) **EP 0 935 975 A1**
(43) Date de publication de la demande: **18.08.1999**
(21) Numéro de dépôt: 99400256.6
(22) Date de dépôt: 04.02.1999
(51) Int. Cl.: A61M 25/00

(54) **Dispositif médical comprenant une tige munie d'un moyen d'absorption des contraintes axiales**

(30) Priorité: 16.02.1998 FR 9801835; 15.04.1998 FR 9804685
(71) Demandeur: B. Braun Celsa, 92100 Boulogne-Billancourt (FR)
(72) Inventeur: Nadal, Guy, 86000 Poitiers (FR); Bovyn, Gilles, 2200 Saint-Brieuc (FR)
(74) Mandataire: Lerner, François

(57) **Abrégé**

L'invention concerne un dispositif (10) à usage médical propre à être introduit dans un conduit (V) d'un corps humain ou animal vivant, ledit dispositif comprenant une tige (20) flexible bio-compatible présentant un axe (xx') le long duquel elle s'étend, hors contrainte, de façon rectiligne, ladite tige (20) ayant longueur selon cet axe (xx'), une extrémité distale (24) et une extrémité proximale (22). Ce dispositif se caractérise en ce que, afin que la tige demeure sensiblement à un endroit déterminé d'implantation dans le conduit (V), elle est munie d'au moins un moyen (30 ; 50 ; 80, 90 ; 72 ; 100 ; 120) d'absorption des contraintes axiales disposé selon son axe (xx') et qui est distinct de celle-ci tout en étant liée à elle, pour que son extrémité proximale (22) ne se déplace sensiblement pas axialement une fois ladite tige implantée à l'endroit déterminé.

## Description

L'invention se rapporte au domaine des dispositifs à usage médical implantable dans un conduit anatomique.

Précisément, il s'agit d'un dispositif comprenant une tige flexible bio-compatible présentant un axe le long duquel elle s'étend, hors contrainte, de façon rectiligne, ladite tige ayant une longueur selon cet axe, une extrémité distale et une extrémité proximale.

Ce type de tige présente actuellement certains inconvénients et ne donne pas entière satisfaction, aussi bien au praticien qui la manipule qu'au patient à l'intérieur du corps duquel elle est placée. En particulier, en cas de mouvement du patient (passage de la position assise à la position couchée, voire à la position foetale, et réciproquement), la tige a tendance à s'adapter à la configuration du conduit (courbure) à l'intérieur duquel elle est introduite et à se déplacer dans celui-ci de telle sorte que ses extrémités ne restent pas en place, ce qui peut être gênant. Il existe donc un problème de stabilité et de maintien en place de ce type de tiges lorsqu'elles sont positionnées à un endroit déterminé d'implantation à l'intérieur du corps d'un patient

Pour résoudre cela, il est proposé que la tige soit munie d'au moins un moyen d'absorption des contraintes axiales disposé selon son axe et qui est distinct de celle-ci tout en étant lié à elle, de sorte que son extrémité proximale ne se déplace sensiblement pas axialement, une fois la tige parvenue à l'endroit déterminé d'implantation dans le conduit

On précise ici que sera « rectiligne » toute tige présentant, au repos, une direction générale d'extension droite suivant son axe et qui ne présente donc pas localement, au moins à l'emplacement de son moyen d'absorption des contraintes, une courbure marquée (sinuosité) pouvant servir d'amortisseur (comme dans n° FR-A-2 715 827).

En relation avec une telle tige, l'invention concerne en particulier les dispositifs pour l'implantation temporaire d'un implant vasculaire, tel qu'un filtre sanguin. Un tel filtre est en particulier décrit dans EP-A-521 522 ou US-A-5 634 942.

L'expérience a montré que lors de l'implantation d'un implant, et en particulier d'un filtre sanguin, il arrive, en raison du flux sanguin, que celui-ci se déplace axialement dans le vaisseau lorsqu'il est dépourvu de moyen d'accrochage à la paroi du vaisseau. On a aussi remarqué que, durant de la période d'implantation temporaire de l'implant, ce dernier peut, suite aux mouvements et changements de position du patient, se déplacer dans le conduit voire s'arracher s'il a commencé à être recouvert d'agrégats cellulaires.

Ainsi, l'invention se rapporte à un dispositif pour la mise en place de façon temporaire à l'intérieur d'un conduit anatomique d'un patient d'un implant comprenant une structure radialement expansible, et en particulier d'un filtre sanguin, ledit dispositif comprenant en particulier la tige telle que présentée ci-avant

Lorsque l'on veut mettre en place l'implant temporairement dans le corps du patient, on peut souhaiter, après avoir introduit la tige à l'intérieur du conduit anatomique du patient, placer sous sa peau l'extrémité proximale de la tige à l'autre bout de laquelle est disposé l'implant. Dans ce cas, l'adaptation de courbure de la tige en cas de mouvement du patient peut aussi provoquer un déplacement douloureux de son extrémité proximale, là où elle sera liée à une pièce sous-cutanée de repérage (voir EP-A-521 222), voire d'une pièce destinée à assurer des opérations d'injection/prélèvement (via une chambre implantable, comme dans FR-A-2 697 995).

Pour résoudre cela, le moyen, ou l'un au moins de ces moyens, d'absorption des contraintes axiales pourra être disposé vers l'extrémité proximale de la tige, là où elle celle-ci est liée à ladite pièce sous-cutanée, laquelle est donc repérable par palpation à travers la peau du patient

Toujours dans le but de résoudre le problème présenté ci-avant, l'invention prévoit en particulier que ladite pièce de repérage soit équipée dudit moyen d'amortissement des contraintes, en particulier sous la forme d'un dispositif à piston et/ou éventuellement ressort

Selon un aspect complémentaire, le moyen d'absorption dès contraintes axiales pourra comprendre un soufflet pouvant s'allonger ou se raccourcir axialement. Cette solution simple garantit une absorption progressive et continue, peut s'adapter aussi bien à une tige pleine que creuse, et peut être positionnée à n'importe quel endroit de celle-ci.

Selon une alternative, le moyen d'absorption des contraintes pourra comprendre un ressort

Selon une autre alternative éventuellement complémentaire des deux précédentes, le moyen d'absorption des contraintes axiales pourra comprendre un piston. Cette solution offre en plus une tenue transversale du moyen d'absorption des contraintes.

En particulier si l'implant est un filtre comprenant une tête de laquelle partent des pattes expansibles radialement, le moyen d'absorption des contraintes axiales pourra être fixé vers l'extrémité distale de la tige. On concentre ainsi l'effet d'amortissement dans une zone critique, c'est-à-dire la zone de pose de l'implant. Ainsi, les contraintes qui s'exercent sur l'implant sont immédiatement compensées par le moyen d'absorption, ces contraintes n'étant alors pas (ou très peu) transmises à la tige.

De façon à résoudre les difficultés que l'on peut avoir à implanter et retirer l'implant, le piston pourra comprendre une seconde tige-poussoir montée coulissante à l'intérieur de la première tige par l'intermédiaire de cette dernière est liée à l'implant.

De façon à améliorer le coulissement relatif du piston à l'intérieur de la tige, le dispositif pourra comprendre en outre un système de guidage et de butée pour limiter son débattement axial à l'intérieur de la tige.

En particulier, ce système pourra comprendre une première bague fixée à l'intérieur de la première tige et dont le diamètre interne est légèrement supérieur au diamètre externe du piston, et seconde une bague (ou protubérance) liée au piston et dont le diamètre externe est légèrement inférieur au diamètre interne de la tige.

Indépendamment des problèmes cités précédemment d'amortissement des contraintes axiales, un autre problème se pose en relation avec l'utilisation d'un site implantable à relier à un dispositif à usage médical propre à être introduit dans un conduit d'un corps humain ou animal vivant, tel qu'un cathéter.

A toutes fins utiles, on notera que l'extrémité «distale» des éléments du dispositif désigne l'extrémité qui doit être implantée le plus profondément dans le corps du patient, l'extrémité «proximale» étant l'extrémité opposée située près de la surface de la peau (voire à l'extérieur).

L'invention et sa mise en oeuvre apparaîtront encore plus clairement à l'aide de la description qui va suivre, faite en référence aux dessins dans lesquels:
- la figure 1 est une vue générale d'un dispositif conforme à l'invention,
- les figures 2 et 3 sont des vues de détail en coupe du dispositif de la figure 1,
- les figures 4 et 5 sont des vues de détail en coupe d'une variante de réalisation du dispositif de la figure 1,
- les figures 6 et 7 sont des vues en coupe d'un détail d'une autre variante de réalisation du dispositif de la figure 1,
- la figure 8 est une vue en coupe schématique du corps d'un patient dans lequel est implanté le dispositif de la figure 1,
- les figures 9 et 10 montrent certains des principaux moyens utilisés pour la mise en place ou le retrait du dispositif de l'invention.
- la figure 11 est une variante de réalisation du dispositif dans laquelle la pièce de repérage sous-cutané est équipée d'un moyen d'amortissement des contraintes axiales,
- la figure 12 est une variante de la figure 11 dans laquelle la pièce de repérage sous-cutané a également une fonction de relais pour un site implantable,
- la figure 13 est une variante de la figure 12 dans laquelle la pièce de repérage sert uniquement de relais et n'est plus munie d'un moyen d'amortissement des contraintes axiales,
- la figure 14 est une variante de la figure 13, et
- la figure 15 est autre variante de réalisation.

L'un des intérêts de l'utilisation de ce dispositif étant en particulier de pouvoir améliorer les conditions d'implantation des filtres sanguins temporaires, on ne décrira ci-après l'invention que dans le cadre d'une telle application, même s'il doit être clair qu'elle peut s'appliquer à d'autres implants médicaux, vasculaires ou non (stents par exemple).

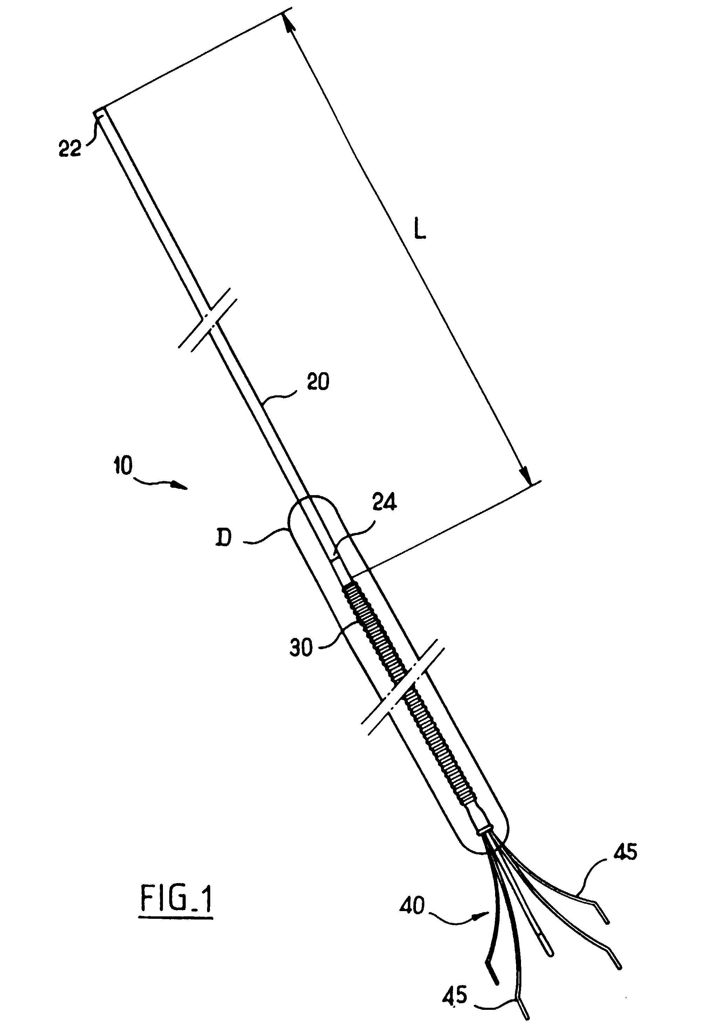

Sur la figure 1 on voit donc illustré dans son ensemble une unité temporaire de filtration 10, d'axe général sensiblement rectiligne xx', pouvant être mise en place par voie percutanée (méthode de « SELDINGER »), ou par dénudation.

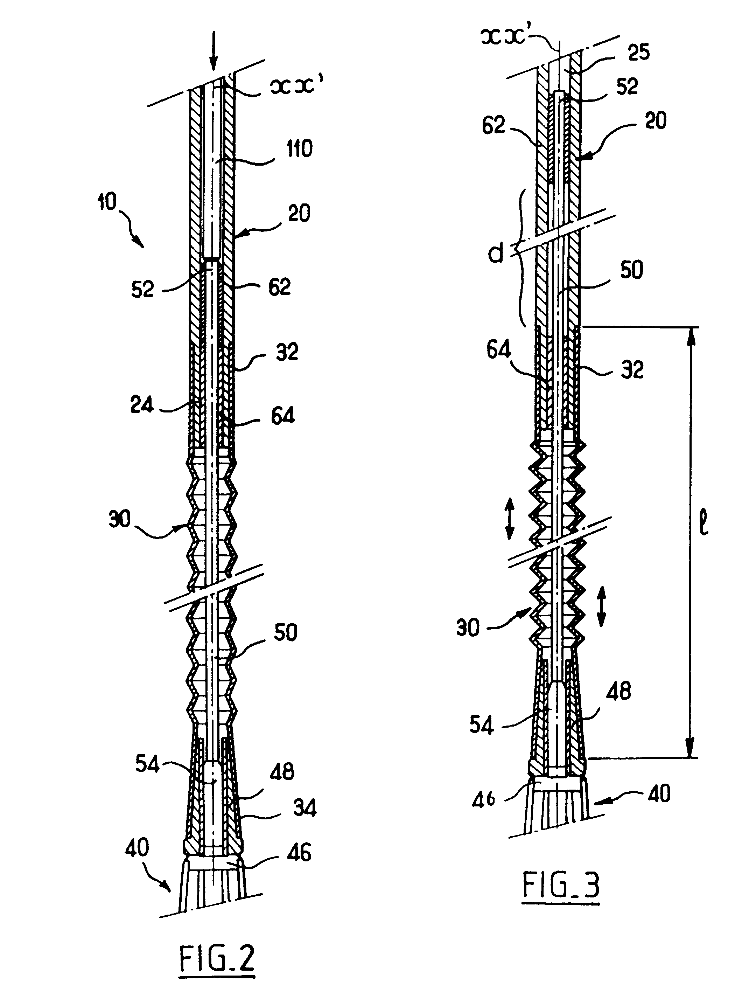

Ce dispositif 10 comprend essentiellement une tige souple 20 rectiligne bio-compatible, d'axe xx' et de longueur L selon cet axe, avec une extrémité proximale 22 et une extrémité distale 24 opposée. Cette tige 20 pourra être pleine ou de préférence présenter un passage intérieur 25 pour former alors un cathéter. Les figures 2 à 5 représentent le détail D de la figure 1.

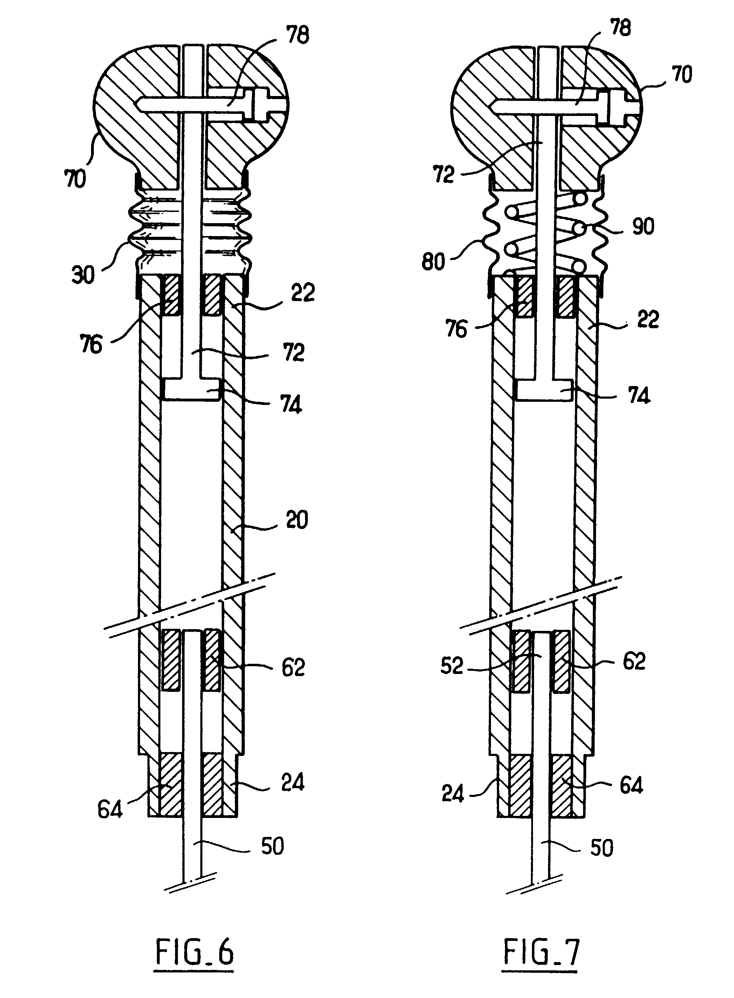

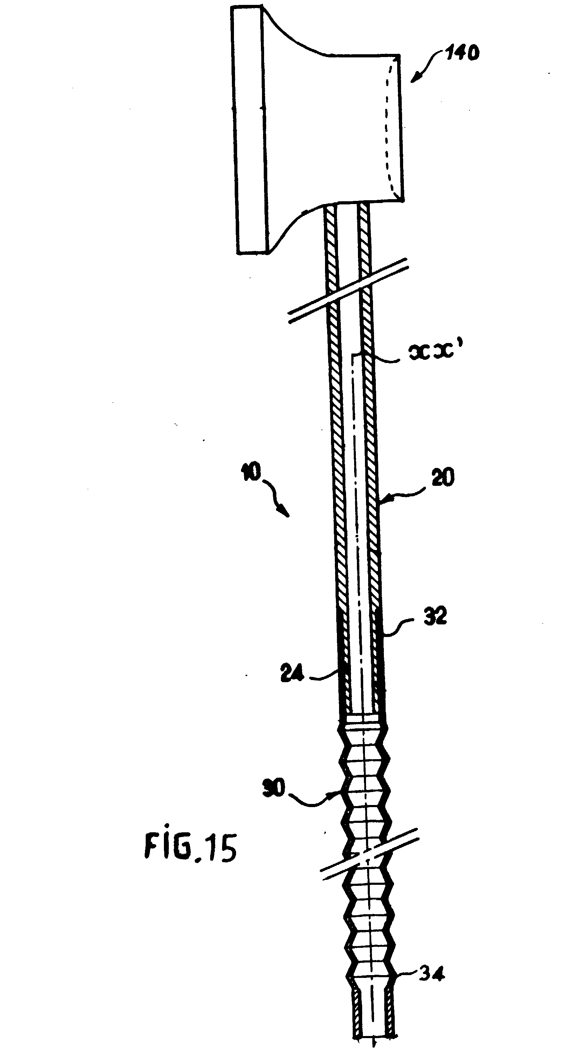

A son extrémité proximale 22, la tige 20 est ici reliée à une pièce 70 sous-cutanée (voir figures 6 et 7) sensiblement fixe en position, appelée aussi « olive de repérage », qui permet, une fois ladite pièce 70 enfouie entièrement sous la peau du patient par exemple en zone sous-clavière, de faciliter le repérage de l'extrémité proximale de la tige 20 par palpation à travers la peau (voir EP-A-211 522). Si la tige 20 est creuse, en alternative, la pièce 70 sous-cutanée pourra être une chambre implantable de distribution d'un produit traitant, telle que décrite dans FR-A-2 697 995. Cette variante de réalisation est illustrée en figure 15.

Le dispositif 10 comprend aussi un soufflet 30 de préférence en silicone, qui présente une extrémité proximale 32 et une extrémité distale 34. Son extrémité proximale 32 est fixée, par exemple par collage ou sertissage, autour de l'extrémité distale 24 de la tige 20. Ce soufflet 30 présente une longueur (l) initiale dans son état libre (état ni comprimé ni étiré axialement) selon l'axe xx' de la tige 20 égale à environ un dixième de la longueur totale L de cette dernière, c'est-à-dire comprise entre environ 5 et 15 centimètres, ce soufflet 30 pouvant s'étendre ou rétrécir axialement jusqu'à environ 50% de sa longueur initiale. De préférence, le soufflet 30 est étanche au liquide et est de type élastiquement déformable, c'est-à-dire qu'après avoir appliqué sur ses deux extrémités une contrainte axiale de compression ou de traction, il revient de lui-même dans sa position de repos où il présente sa longueur (1) initiale.

A son extrémité distale 34, le soufflet 30 est aussi fixé à un filtre sanguin 40 en métal bio-compatible (acier inoxydable) qui présente, en position déployée, une forme générale de corolle conique. Ce filtre 40 comprend des pattes 45 dépourvues de tout moyen d'accrochage (crochet par exemple) au vaisseau et toutes issues d'une tête 46 commune autour de laquelle est fixée le soufflet 30 par l'intermédiaire d'une bague 48. Ces pattes 45 pourront en particulier être auto-expansibles radialement, hors contraintes, pour avoir naturellement tendance à se déployer jusqu'à leur extrémité libre vienne au contact de la paroi du vaisseau.

Le dispositif 10 comprend également une seconde tige 50, faisant office de piston et de poussoir, disposée à l'intérieur de la tige 20 de façon à pouvoir coulisser longitudinalement à l'intérieur de celle-ci. Cette tige-poussoir 50 présente une extrémité distale 54 fixée à la tête 46 du filtre 40, et une extrémité proximale 52 autour de laquelle est sertie une première bague 62 coopérant avec une seconde bague 64 fixée à l'intérieur de la tige 20, à son extrémité distale 34. Ces deux bagues 62 et 64 servent à guider la tige-poussoir 50 à l'intérieur de la tige 20 et à limiter son débattement (d) axial en faisant office de butée lorsque le soufflet 30 se comprime ou s'étend.

Le filtre 40 étant destiné à être implanté à l'intérieur d'un vaisseau sanguin, on a constaté, dans les dispositifs de l'art antérieur, qu'il était particulièrement soumis à des contraintes mécaniques occasionnées par les mouvements du patient. Or de telles contraintes tendent à se propager le long de la tige 20. La pièce 70 de repérage, de même que le filtre 20 lui-même, peuvent donc être soumis à un déplacement, ce qui est peut confortable pour le patient et risque d'entraîner des complications annexes.

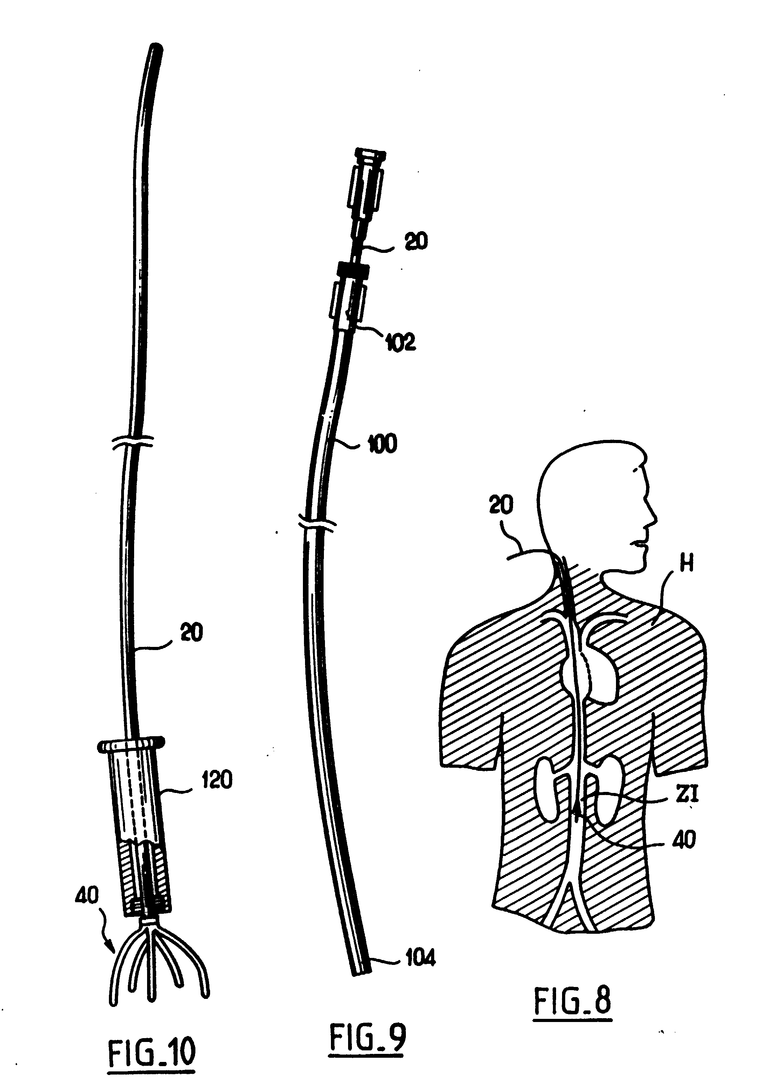

Ainsi, le soufflet 30 permet d'absorber ces contraintes axiales et de compenser des déplacements intempestifs du filtre ou de la pièce 70 de repérage. Comme on peut le voir sur la figure 3, où l'on considère que le filtre est implanté dans le vaisseau (voir aussi figure 8), le soufflet 30 est dans un état libre dans lequel le poussoir 50 peut se déplacer axialement. Il peut donc s'étirer ou se comprimer axialement au gré des mouvements du patient, et ce sans que le filtre 40 ou l'extrémité proximale 22 de la tige 20 ne se déplace axialement, en particulier lorsque le filtre 40 est recouvert d'un agrégat cellulaire ou lorsqu'il est partiellement coincé. Le déplacement du soufflet 30 entraîne évidemment celui de la tige-poussoir 50 à l'intérieur de la tige 20, ce qui contribue à améliorer l'absorption des contraintes axiales en évitant qu'elles se transmettent à la première tige 20. On peut donc noter que cette seconde tige-poussoir 50 constitue en elle-même un moyen d'absorption des contraintes axiales, sous la forme d'un piston.

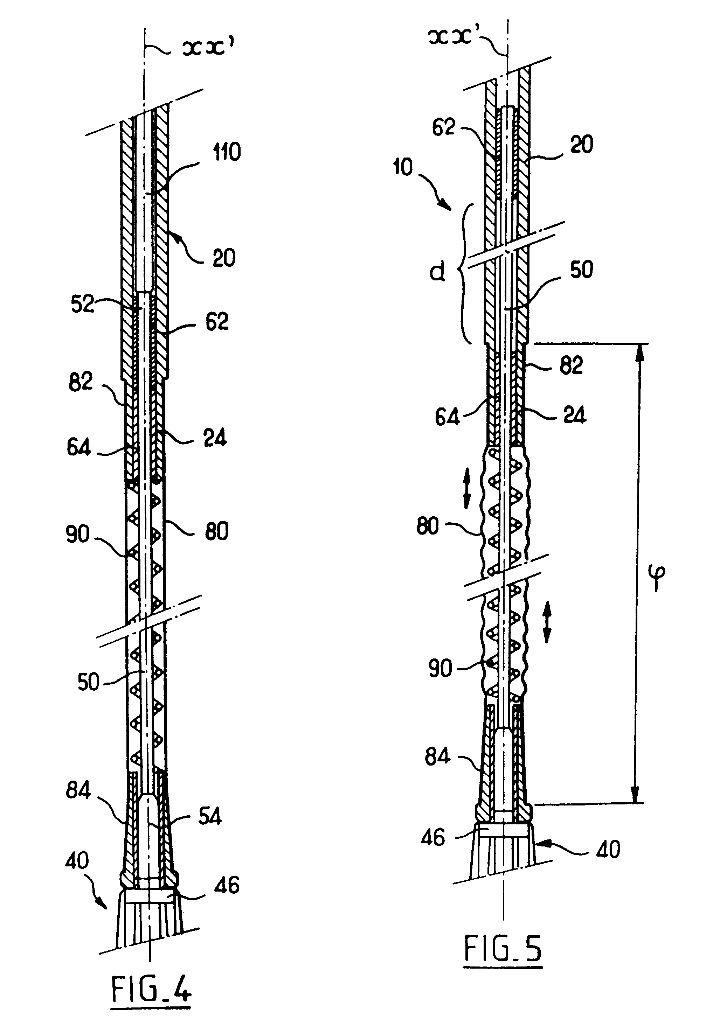

Les figures 4 et 5 montrent une variante de réalisation du dispositif des figures 2 et 3 utilisable à l'une et/ou l'autre des extrémités de la tige 20. Dans cette solution, le moyen d'absorption des contraintes comprend une enveloppe 80 (ou un manchon) étanche, réalisée dans un matériau bio-compatible très fin pouvant en particulier se déformer et se plier sur lui-même sans se percer, tel que du silicone, et un ressort hélicoïdal 90. L'enveloppe 80 est fixée, comme le soufflet 30, à la tête 46 du filtre 40 d'une part et à l'extrémité distale 24 de la tige 20 d'autre part, de façon à rendre le dispositif 10 étanche au fluide circulant dans le vaisseau. Le ressort 90 est monté à l'intérieur de l'enveloppe 80 et entoure la tige-poussoir 50, laquelle est munie d'une bague 62. Ce ressort 90 prend appui, dans un état au moins partiellement comprimé axialement (en particulier lors de l'implantation comme illustré sur la figure 4), d'une part contre l'extrémité distale 24 de la tige 20 et d'autre part contre le prolongement 48 de la tête du filtre. L'ensemble comprenant l'enveloppe 80 et le ressort 90 ont ainsi la même fonction d'absorption des contraintes que le soufflet 30 ou la tige-poussoir 50 présentés précédemment

Les figures 6 et 7 montrent une autre variante de réalisation, utilisable de préférence en collaboration avec l'une des deux présentée ci-avant et disposée à l'extrémité proximale de la tige 20, à l'endroit où se trouve la pièce 70 de repérage. Dans cette solution, la pièce de repérage 70 est montée sur un tube 72, plein ou creux, qui pénètre à l'intérieur de la tige 20 sur une certaine longueur, par exemple comprise entre environ 2 et 12 centimètres. Ce tube 72, faisant office de piston, comprend un système de guidage axial et de butée comprenant une excroissance 74 (ou une première bague) liée à son extrémité distale et dont le diamètre externe est légèrement inférieur au diamètre interne de la tige 20 coopérant avec une seconde bague 76 sertie à l'intérieur de l'extrémité proximale de la tige 20 et dont le diamètre interne est légèrement supérieur au diamètre externe du tube 72. Enfin, un soufflet 30 étanche relie l'extrémité proximale 22 de la tige 20 à la pièce 70 de repérage, laquelle est maintenue à la tige 20, par exemple par un clou 78.

Sur la figure 7, on a remplacé le soufflet 30 par une combinaison comprenant un ressort hélicoïdal 90 et une enveloppe 80 souple et étanche ayant la même fonction.

Dans ces deux variantes, les contraintes axiales qui s'exercent sur l'extrémité distale 24 de la tige 20 et qui parcourent celle-ci jusqu'à son extrémité proximale 22 sont absorbées par le piston 72 et par le soufflet 30 ou le ressort 90. Ainsi, la pièce 70 de repérage sous-cutané ne se déplace pas sous la peau du patient

L'implantation jusqu'à la zone d'implantation (ZI) (ou le retrait) de l'unité 40 de filtration sanguine s'effectue comme dans FR-A-2 715 827, par voie endoluminale percutanée, si nécessaire par l'intermédiaire d'une gaine introductrice 100.

Une fois la gaine 100 en position, elle sert de guide pour la mise en place du filtre 40, lequel est habituellement préconditionné à l'état radialement restreint de ses pattes dans une sorte de seringue 120 de conditionnement (non représentée) vissée sur l'embout proximal 102 de la gaine qui débouche hors du corps du patient

Pour faire descendre l'ensemble comprenant la tige 20, le filtre 40 solidaire de sa tige-poussoir 50 et le soufflet 30, le praticien agit sur la tige-poussoir 50 par l'extérieur du corps du patient à l'aide d'un mandrin 110 (tige de préférence pleine et plus rigide que la tige). Le soufflet 30 (ou l'enveloppe 80) s'étire alors axialement tandis que le filtre 40 se libère et s'expanse radialement, hors de la gaine 100. Le praticien retire alors le mandrin 110, coupe la tige 20 à longueur et vient fixer à son extrémité proximale 12 la pièce 70 à l'aide du clou 78 avant d'enfouir le tout dans les tissus du patient et de refermer la voie d'accès.

Une fois le filtre 40 en place, le moyen d'absorption des contraintes axiales (piston, soufflet, ressort) remplit sa fonction et empêche au filtre et/ou à la pièce 70 de se déplacer axialement à l'intérieur du vaisseau, en particulier lorsque le patient change de position.

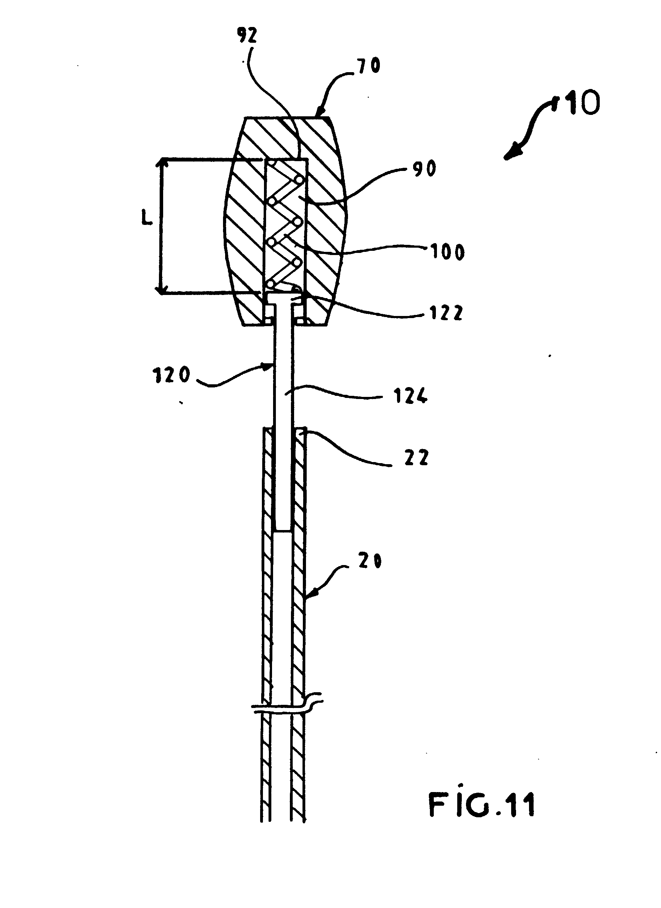

Pour des questions de compréhension, les échelles des figures 11 à 14 qui suivent n'ont pas été respectées

Une autre variante de réalisation est représentée sur la figure 11 où seulement l'extrémité proximale de la tige 20 est dessinée. La pièce 70 de repérage sous-cutané, de préférence en silicone, présente une cavité interne 90 à l'intérieur de laquelle se trouve un ressort 100 de compression prenant contre le fond 92 de la cavité 90 et sur lequel repose un piston 120 en métal biocompatible (acier inoxydable) ou matériau plastique comprenant une tête 122 sensiblement en forme de disque et une tige 124, de préférence pleine. Cette tige 124 de piston est fixée à l'extrémité proximale 22 de la tige 20 porteuse du filtre 40, par exemple par sertissage, ou collage ou toute autre technique connue. Le ressort 100 peut se comprimer axialement suivant une course E. Ainsi, lorsque le dispositif 10 est implanté dans le corps du patient et que la pièce 70 de repérage sous-cutané est disposée sous la peau, par exemple en zone sous-clavière, le ressort 100 absorbe élastiquement les contraintes axiales pouvant survenir en s'écrasant, comme cela a déjà été exposé précédemment Ainsi, la pièce 70 de repérage et le filtre 40 peuvent demeurer à leur endroit respectif d'implantation.

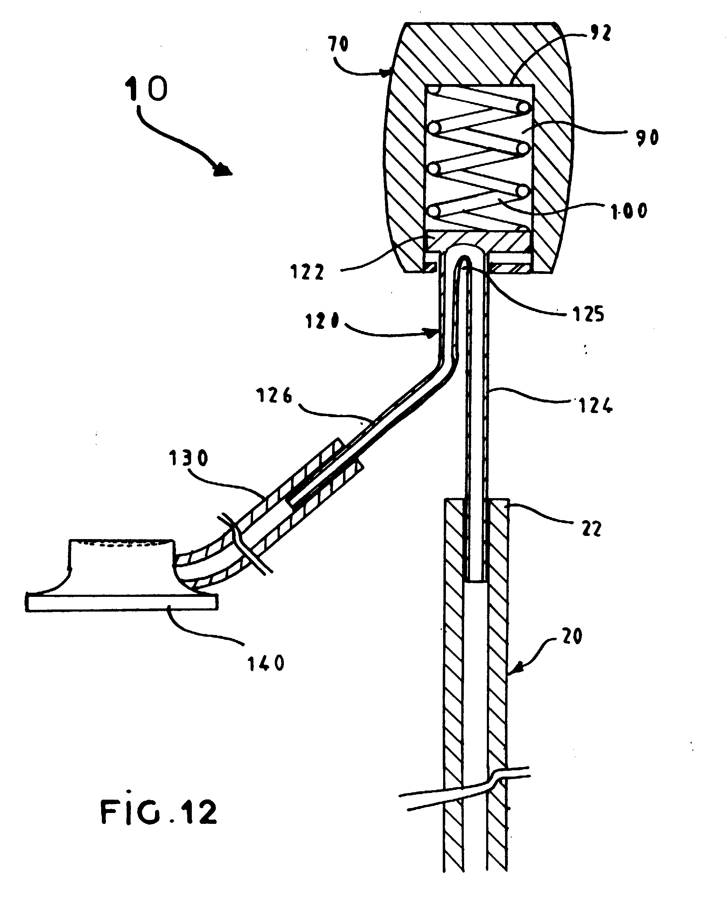

Sur la figure 12, la pièce 70 de repérage comprend toujours une cavité interne 90 à l'intérieur de laquelle est logée un ressort 100 sur lequel repose un piston 120 relié, par l'intermédiaire de sa tige 124, à la tige 20 porteuse du filtre 40 de la même façon que précédemment La tige 124 du piston est creuse et forme un coude 125 (courbure prononcée) à l'endroit de la tête 122 du piston. Cette tige 124 est liée, par une « branche » 126, à un cathéter 130 souple, lequel cathéter est implanté sous la peau du patient et est connecté à un site implantable 140 (appelé aussi chambre implantable) de type connu (voir brevet FR-A-2 697 995) également situé sous la peau par au niveau de la cage thoracique. Ce site implantable 140 est adapté pour que l'on puisse piquer dans sa paroi 142 auto-obturante (par exemple en silicone) une seringue contenant un produit traitant à acheminer à l'intérieur du vaisseau jusqu'au filtre, dans l'optique par exemple de détruire un caillot sanguin capturé. Ainsi, la pièce 70 de repérage sous-cutané, en plus de comprendre un moyen d'amortissement des contraintes axiales, sert de relais à un site implantable. Cette solution évite d'avoir à fixer le site implantable 140 directement à l'extrémité proximale 22 de la tige porteuse 20 (ce qui ne permet pas d'amortir les contraintes axiales) tout en procurant un confort amélioré au patient

Dans ces deux variantes de réalisation, le moyen d'amortissement des contraintes axiales peut être autre qu'un ressort 100 de compression, par exemple un ressort à lame, voire un dispositif d'amortissement par fluide (air ou liquide), auquel cas le piston sera monté de façon étanche dans la cavité.

On notera que l'utilisation d'une tige flexible biocompatible telle que représentée sur les figures 1 à 12 peut se faire sans la présence d'un quelconque implant à son extrémité distale 22. Ainsi, comme cela est illustré sur la figure 15, il est possible de relier un cathéter 20 d'injection à une chambre implantable 140 (identique à celle de la figure 12) fixée sous la peau du patient et de munir ledit cathéter 20 d'un soufflet 30. Les changements de configurations du vaisseau dans lequel le cathéter est implanté sont compensés par les moyens d'amortissement 30, permettant ainsi au cathéter de rester positionné à un endroit déterminé d'implantation à l'intérieur du conduit du patient Ainsi, son extrémité proximale 22 ne se déplace sensiblement pas axialement, ceci étant d'autant plus vrai si cette extrémité proximale de la tige flexible concernée est directement fixée à la peau du patient ou si elle est pourvue d'un dispositif de repérage sous-cutané tel que l'olive des figures 6 et 7 ou 11 et 12.

Indépendamment des problèmes résolus par les dispositifs présentés sur les figures 1 à 12 et 15 en relation avec l'amortissement des contraintes axiales, se pose un problème distinct relatif à l'utilisation d'un site implantable tel que décrit dans le brevet FR-A-2 697 995. En effet, il peut y avoir un problème pour relier un tel site implantable à l'extrémité proximale 22 de la tige 20, en particulier lorsque l'on souhaite pouvoir repérer cette extrémité proximale 22 sous la peau du patient tout en assurant une bonne assise audit site.

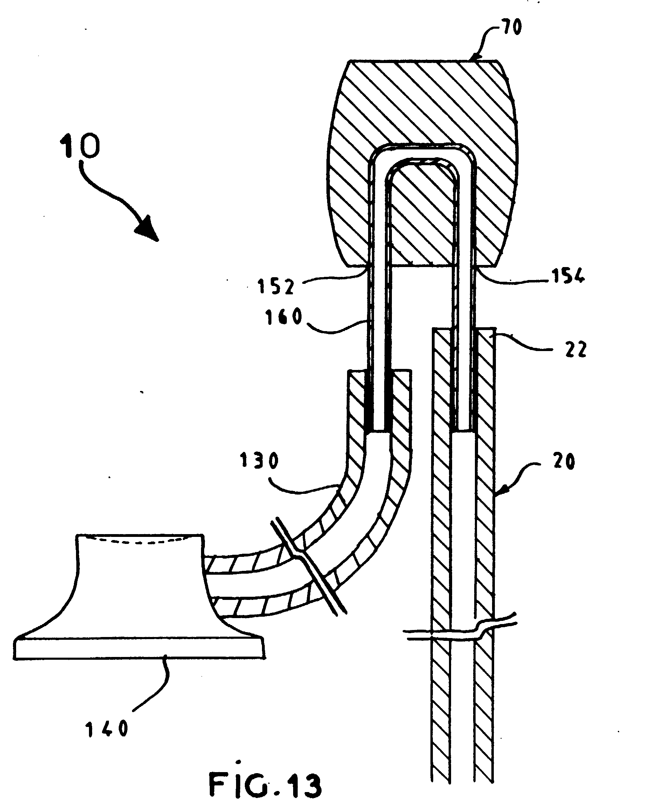

La figure 13 présente une solution à ce problème spécifique. Dans cette solution, la pièce 70 de repérage sous-cutané comporte un canal 150 interne en forme de « U » (ou de « L ») débouchant en deux orifices 152 et 154 disposés de préférence sur une même face de la pièce 70, le «U» pouvant être plus ou moins ouvert. Un tube 160 métallique creux est inséré dans ce canal 150 et sort par les deux orifices, d'un côté pour être connecté à la tige porteuse 20, et de l'autre côté pour être connecté à un cathéter souple 130 de perfusion placé sous la peau du patient et relié à un site implantable 140, comme dans la figure 12. La pièce 70 de repérage ainsi représentée est donc dépourvue de moyen d'amortissement des contraintes et fait uniquement office de relais de façon à permettre le transit d'un produit de traitement entre l'extrémité distale 24 de la tige porteuse 20 et le site implantable 140.

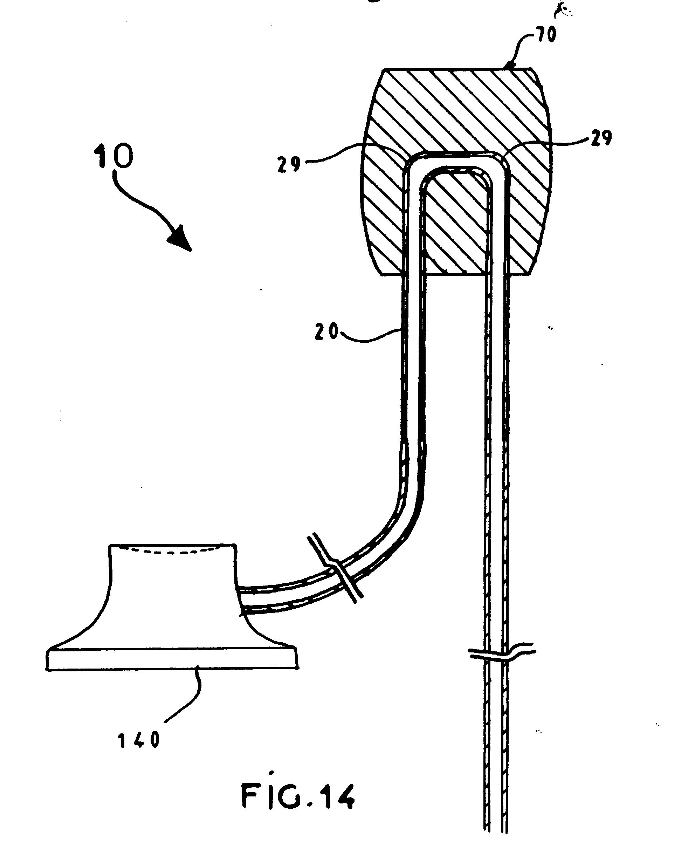

Selon une alternative représentée sur la figure 14, la tige porteuse 20 creuse peut être reliée directement au site implantable 140 tout en transitant par la pièce 70 de repérage. Cette solution évite les connexions entre les différentes parties creuses (tige du piston, cathéter souple, tige porteuse), ces connexions pouvant céder ou fuir et être sources de problèmes. Ainsi, la tige porteuse 20 présente une ou plusieurs courbure(s) 29 (coude) au niveau de la pièce de repérage. Pour éviter les phénomènes de plicatures, cette tige porteuse 20 est de préférence insérée dans un manchon (non représenté) ou de préférence directement dans une cavité préformée à l'intérieur de la pièce de repérage de façon à lui imposer la courbure souhaitée. Ainsi, il est toujours possible d'injecter un produit du site implantable 140 jusqu'à l'extrémité distale 24 de la tige porteuse 20 tout en conservant une pièce 70 de repérage utilisable lorsque l'on veut retirer du corps du patient le filtre 140 implanté temporairement.

Bien entendu, l'invention n'est nullement limitée aux modes de réalisation présentés ci-dessus.

Ainsi, on pourra prévoir que le filtre 40 soit directement fixé à l'extrémité distale 24 de la tige 20 (donc sans poussoir) et disposer le soufflet 30 ou l'ensemble ressort 80/enveloppe 90 en partie médiane de la tige 20, laquelle sera donc en deux tronçons séparés par le « moyen d'absorption ».

On peut également envisager d'utiliser ce dispositif dans d'autres conduits tels que l'oesophage ou la trachée artère, ou avec d'autres types d'implants (stent).

Les différents moyens d'amortissement des contraintes axiales peuvent indifféremment être combinés entre eux. En particulier, la solution à soufflet (ou ressort) disposé à l'extrémité distale de la tige 20 peut être combinée avec une pièce 70 de repérage sous-cutané munie d'un moyen d'absorption des contraintes axiales.

Enfin, le soufflet 30 (voire éventuellement l'enveloppe souple 80) peut être muni d'un système de guidage axial, tel par exemple qu'une série de bagues en matière plastique ou en métal, serties à l'intérieur du soufflet et régulièrement espacées le long de la tige-poussoir 50 autour de laquelle elles peuvent coulisser. Ainsi, le soufflet 30 ne viendra pas frotter contre la tige-poussoir en se courbant (fléchissement de type flambage) lors de sa compression, ce qui évitera une dégradation prématurée (usure) de celui-ci et/ou un phénomène de grippage pouvant gêner l'absorption des contraintes voire déplacer le filtre.

## Revendications

1. Dispositif (10) à usage médical propre à être introduit dans un conduit (V) d'un corps humain ou animal vivant, ledit dispositif comprenant une tige (20) flexible bio-compatible présentant un axe (xx') le long duquel elle s'étend, hors contrainte, de façon rectiligne, ladite tige (20) ayant une longueur (L) selon cet axe (xx'), une extrémité distale (24) et une extrémité proximale (22), caractérisé en ce que, afin que la tige (20) demeure sensiblement à un endroit déterminé d'implantation dans le conduit (V), elle est munie d'au moins un moyen (30 ; 50 ; 80, 90 ; 72; 100; 120) d'absorption des contraintes axiales disposé selon son axe (xx') et qui est distinct de celle-ci tout en étant liée à elle, pour que son extrémité proximale (22) ne se déplace sensiblement pas axialement, une fois la tige (20) implantée à l'endroit déterminé.

2. Dispositif (10) à usage médical propre à être introduit de façon temporaire dans un conduit (V) d'un corps humain ou animal vivant, ledit dispositif (10) comprenant:
- une tige (20) flexible bio-compatible présentant un axe (xx') le long duquel elle s'étend, hors contrainte, de façon rectiligne, ladite tige (20) ayant longueur (L) selon cet axe (xx'), une extrémité distale (24) et une extrémité proximale (22), et
- un filtre sanguin (40) lié à l'extrémité distale (12) de la tige (20) et comprenant une structure (45) radialement expansible,
caractérisé en ce que, afin que la tige (20) et le filtre (40) demeurent sensiblement à un endroit déterminé d'implantation dans le conduit (V), ladite tige (20) est munie d'au moins un moyen (30; 50 ; 80, 90 ; 72; 100 ; 120) d'absorption des contraintes axiales disposé selon son axe (xx') et qui est distinct de celle-ci tout en étant liée à elle, pour que son extrémité proximale (22) ne se déplace sensiblement pas axialement, une fois la tige (20) et le filtre (40) implantés à l'endroit déterminé.

3. Dispositif (10) selon l'une quelconque des revendications précédentes, caractérisé en ce que le moyen (30 ; 50 ; 80, 90 ; 72; 100 ; 120) d'absorption des contraintes axiales (ou l'un au moins de ces moyens) est disposé vers l'extrémité proximale (22) de la tige (20), où celle-ci est liée à une pièce (70) sous-cutanée repérable par palpation à travers la peau du patient.

4. Dispositif (10) selon la revendication 3, caractérisé en ce que la pièce (70) sous-cutanée repérable par palpation à travers la peau du patient est équipée dudit moyen d'absorption (100 ; 120) des contraintes axiales.

5. Dispositif (10) selon l'une quelconque des revendications 1 à 3, caractérisé en ce que le moyen (30; 50; 72) d'absorption des contraintes axiales comprend un soufflet (30) pouvant s'allonger ou se raccourcir axialement.

6. Dispositif (10) selon l'une quelconque des revendications 1 à 4, caractérisé en ce que le moyen (80, 90; 72; 100; 120) d'absorption des contraintes axiales comprend un ressort (80; 100) hélicoïdal.

7. Dispositif (10) selon l'une quelconque des revendications précédentes, caractérisé en ce que le moyen (30; 50; 80, 90; 72 ;100; 120) d'absorption des contraintes axiales comprend un piston (50; 72; 120).

8. Dispositif selon l'une quelconque des revendications précédentes, caractérisé en ce que le moyen (30 ; 50; 80, 90; 72 ;100 ; 120) d'absorption des contraintes axiales (l'un au moins de ces moyens) est disposé vers l'extrémité distale (24) de la tige (20).

9. Dispositif selon les revendications 7 et 8, caractérisé en ce que le piston (50; 72) comprend une seconde tige-poussoir (50) montée coulissante à l'intérieur de la première tige (20) et par l'intermédiaire de laquelle l'implant (40) est lié à la tige (20).

10. Dispositif (10) selon la revendication 7 ou 9, caractérisé en ce qu'il comprend en outre un système (62, 64; 74, 76) de guidage et de butée du piston (50; 72) pour limiter son débattement axial à l'intérieur de la première tige (20).

11. Dispositif (10) selon la revendication 10, caractérisé en ce que le système (62, 64; 74, 76) de guidage et de butée comprend :
- une première bague (62; 74 ) liée au piston (50; 72) et dont le diamètre externe est légèrement inférieur au diamètre interne de la tige (20), et
- une seconde bague (64; 76) fixée à l'intérieur de la tige (20) et dont le diamètre interne est légèrement supérieur au diamètre externe du piston (50 ; 72).
